# EUROPEAN PATENT APPLICATION

(11) **EP 2 343 089 A1**
(43) Date of publication of application: **13.07.2011**
(21) Application number: 09815698.7
(22) Date of filing: 11.09.2009
(51) Int. Cl.: A61K 47/26, A61K 31/4709, A23K 1/17, A23K 1/18

(54) **UTILIZATION OF XYLITOL OR ITS DERIVATIVES FOR TASTE-MASKING CHEMOTHERAPY DRUGS OF THE QUINOLONE-O-NAPHTHYRIDONE CARBOXYLIC ACID GROUP ADMINISTRATED IN FOOD INTENDED FOR PIGS**

(30) Priority: 23.09.2008 ES 200802691; 27.07.2009 ES 200901651
(71) Applicant: Laboratorio Jaer, S.a., Barcelona (ES)
(72) Inventor: MILAN MESA, Manuel, 08620 Sant Vincenç de Horts Barcelona (ES); MARTÍ SANROMA, Gabriel, 08620 Sant Vincenç de Horts Barcelona (ES); DE COZAR GARCIA, Sergio, 08620 Sant Vincenç de Horts Barcelona (ES)
(74) Representative: Maldonado Jordan, Julia
(86) International application number: PCT/ES2009/000448
(87) International publication number: WO 2010/034853

(57) **Abstract**

Utilization of xylitol or its derivatives for taste-masking chemotherapy drugs of the quinolone-o-naftiridon carboxylic acid group especially enrofloxacin, administered in food intended for pigs including water. The xylitol may be used alone or together with other synergists, in particular arginine. The xylitol is in a proportion by weight lying between 1 and 100 parts of xylitol to 1 part of quinolone, and more particularly between 20 and 70 parts of xylitol to 1 part of chemotherapy drug. Arginine, when present, is in a proportion by weight lying between 0.5 to 2 parts of arginine to 1 part of chemotherapy drug. The utilization of xylitol furthermore comprises combination with other synergists of xylitol or its derivates, not being arginine, to increase the masking effect of the xylitol.

## Description

### Object of the Invention

This present invention refers to the utilization of xylitol or a derivative of same on its own or together with arginine for the normalisation of the voluntary intake, by pigs, of feeds that contain therapeutic doses of chemotherapy drugs of the quinolone-o-naftiridon carboxylic acid group, especially enrofloxacin, as these chemotherapy drugs are found to be very bitter by the animals. Any substance with nutritional value, including water, is to be understood as foodstuff.

### Background to the Invention

The sensation of sweet or bitter flavours of the substances is very different between mammals (Sato T and Bedler LM. 1997 Broad tuning of rat taste cells to four basic taste stimuli. Chem. Senses; 22:287-293) *(*Glaser, D., Tinti, J.M., Nofre, C., 1998 Taste preference in non-human primates to compounds sweet in man. Ann. N. Y. Acad. Sci., Nov. 30; 855:169) *(*Jakinovich, W Jr and Sugarman, D. 1988 Sugar taste reception in mammals. Chem. Senses, 13, 13-31*) (*Hellekant, G. 1976b On the gustatory effects of monellin and thaumatin in dog, hamster, pig and rabbit. Chem. Sens. Flavor, 2, 97-105*).*

As a result of these differences, the remedies to conceal the bitter taste in humans, for example by means of blocking the receptors, cannot obviously be extrapolated to the rest of the mammals, and in fact such remedies are normally ineffective in the other species.

In so far as refers to the pig species different studies have been carried out, both bio-medical *(*Chamorro, de Paz, Fernandez, and Anel, 1993, Fungiform papillae of the pig and the wild boar analysed by scanning electron microscopy. Scan. Microsc., 7, 313 - 322) *(*Miller and Reedy, 1990. Variations in Human Taste Bud Density and Taste Intensity Perception. Physiology & Behaviour, 47: 1213-1219*) (*Danilova, V., Roberts, T., Hellekant, G. 1999 Responses of single taste fibers and whole chorda tympani and glossopharyngeal nerve in the domestic pig, wild boar*) and equally based on batteries of organoleptic trials aimed at determining the taste preferences (*Glaser, D., Wanner, M., Tinti, J. M., Nofre, C. 2000 Gustatory responses of pigs to various natural and artificial compounds known to be sweet in man. Food Chem, 68, 2000 375-385), from which it results that pigs have a number of gustatory papillae of between 3 and 4 times greater than the number that the human species have and the gustatory response in pigs and humans to sweeteners, bitters or mixtures of both do not demonstrate a clear and predictable relationship either as a result of the density of the gustatory papillae in each species, or as a result of the phylogenetic proximity of the species, or as a result of the chemical structure of the product *(*Glaser, D. 2002 specialization and phyletic trends of sweetness reception in animals. Pure App. Chem., 74, 2002, 1153-1158*).*

These considerations have to be taken into account together with the fact of the abundant veterinary use of bitter substances, for example the chemotherapy drugs belonging to the quinolone-o-naftiridon carboxylic acid group, commonly known, and quoted here as quinolones. The quinolones are cheap chemotherapy drugs of synthetic origin and very bitter, having a quick and very effective bactericide effect, the most well known of which are nalyxidic acid, enrofloxacin, danofloxacine, marbofloxacine, sarafloxacine, ciprofloxacine, oxifloxacine, moxifloxacine and levofloxacine.

The preference for enrofloxacin, danafloxacine and sarafloxacine has grown significantly in veterinary therapeutics as a result of its greater effectiveness when compared with other veterinary use chemical therapies, such as the associations of sulphadiazine-trimethoprim, cephalosporine, betalactamides, macrolides, tetracyclines or phenicols *(*Salmon, S.A., Watts, J.L., Case, C.A., Hoffman, L.J., Wegener, H.C., and Yancey, R.J. Jr. 1995 Comparison of MICs of ceftiofur and other antimicrobials agents against bacterial pathogens of swine from the United States, Canada and Demank. J. Clin. Microbiol, 33(9), 2435-2444).

The enrofloxacin stands out on being especially effective in pigs against diseases caused by such frequent agents like *Escherichia coli, Actinobacillus pleuropneumoniae, Salmonellae, Pasteurella multocida and Micoplasma pleuropneumoniae.*

But the problem of the quinolones, for example enrofloxacin, is that such chemotherapy drugs in therapeutic concentrations are so intensely bitter, that all attempts at their oral administration with the feed have brought about the total and absolute rejection by pigs, whose reaction to bitter tastes is significantly more intense than in humans.

This disadvantage forces the application of the quinolones in pigs to be carried out by parenteral route of administration; hence its administration is very inconvenient and costly in time and effort. No current technique has solved this restriction in a satisfactory manner.

Document W02006013416 describes the blocking of the taste receptors against a bitter substance by means of mixing it with a product that competes with the same receptors, and even which presents a greater affinity for them.

Other documents claim different solutions based on the reduction of the solubility of such chemical therapies, for the purpose of preventing the ionisation of same on coming into contact with the saliva, and therefore making the coming into contact with the taste receptors impossible.

Hence, the document EP1411894 describes the use of ionic exchange resins with which the solubility of said chemotherapy drugs is reduced. Documents EP1355629 and EP0855183 describe formulations that remove the bitter sensation caused by nalidyxic acid derivatives by means of making solid dispersions, using insoluble matrixes. Document WO03077842 describes the formation of a suspension with quinolone derivatives that reduce their solubility until these are in the digestive system. Document EP1641439 describes a formulation for tablets, with differing flavourings on the basis of meat extracts, with which an acceptable palatability is obtained for the animals.

All of the stated solutions allow the administration of bitter chemotherapy drugs by mouth, but only in the case solid forms or dispersions. None of these allows the administration to be made dissolved in drinking water, as in that case the chemotherapy drug must be soluble in an aqueous medium and the remedies proposed cannot thus conceal the intense bitterness of the quinolones.

It must be highlighted at this point that the administration of the quinolones dissolved in drinking water is much cheaper than the individual treatment of each animal. As a solution to the problem for the oral administration in the drinking water, document ES2213829T offers the mixtures of nalidyxic acid derivatives with embonic acid or its water soluble salts. But the mixing of quinolones with water soluble salts of embonic acid results in a significant amount of corresponding quinolone embonate, which as such is basically insoluble and therefore insipid but also ineffective for the administration in the drinking water, into which the quinolone has to necessarily be dissolved. In addition, even though the document states that the administration of such a mixture inhibits the bitter sensation of the quinolone, precisely due to the greater part of said quinolone is insoluble, there remains sufficient free quinolone so as to make the refusal of the animal to drink on the farm continue to be a significant problem.

The quinolones are also used in human therapy, where the attempts to help the palatability of the oral formulations are the source of several patented procedures. However, in these cases, the circumstances are radically different to those found in veterinary therapeutics. The administration in humans is made at high concentrations, hence there is a reduced volume, that can reasonably go from a dessert spoon (5 ml) to a glass of water (200 ml) and the volume fixed is swallowed in one go in a time that is less than one minute. In this case an attempt is being made to make an aware ingestion more pleasant, which makes multiple strategies and a variety of formulations possible, all in line with the previously stated documents.

But these strategies for humans are ruled out in the veterinary problem solution, if the administration of the quinolone is wanted in a convenient and cheap method such as being dissolved in water. Typically, on a daily basis, a pig consumes some 8% of its weight in water. The failure to ingest this amount of water brings about the appearance of serious problems for breeding, such as the failure to increase weight and even the death of the animal. All of the attempts currently known to include a therapeutic dose of any quinolone in the drinking water have been negative, in general complete refusal of the animals to drink the water. Not even with prolonged thirst will the animals drink. The consequent result is inevitable and manifests itself with the above stated serious problems.

### Description of the Invention

This problem of the palatability of the drinking water in pigs is solved by means of the present invention, hence it is revealed that xylitol (2R, 3R, 4S) - pentano-1, 2, 3, 4, 5 -ol, or its derivatives produce an unexpected and non obvious effect of strongly masking the bitter taste of the feeds in the pig species, the drinking water being included in this concept.

The non obvious effect discovered by the inventors, which has been applied in the invention, is that the xylitol in pigs has a high level of concealment of bitter tastes, not proportional to its sweetening power: Very moderate amounts of xylitol dissolved in the drinking water of pigs will be sufficient to completely and satisfactorily conceal the presence of therapeutic amounts of quinolones that are dissolved in the same water. With the presence of xylitol, the pigs daily ingest the amounts of water necessary in spite of the fact that they are treated with the medically necessary amounts of quinolones.

The presence of moderate amounts of xylitol in the animal feed allows the quinolones to be medicinally administered in the feed.

Thus, as the preference of pigs towards food such as water that contains natural or artificial sweeteners for the food itself is well known *(Glaser, D., Wanner, M., Tinti, J.M., Nofre, loc. cit.),* it is not obvious that xylitol specifically, or its derivatives, in reduced doses produce a strong masking effect for intense bitter tastes, such as those of the quinolone family or for example enrofloxacin, up to a point that the animal feed or water containing said very bitter substances in therapeutic doses are ingested by breeding and adult animals without the animals showing any reaction different to that of untreated water.

The xylitol belongs to the alcohol sugar group, substances whose general formula is CₙOₙH(₂ₙ₊₂), examples of which are: erythritol (n=4), arabitol (n=5), manitol (n=6), or sorbitol (n=6), or the polysaccharides of these, the isomaltose (disacharide made up of glucose and manitol) or maltitol (disacharide made from glucose and sorbitol). None of these other alcohol sugars show the masking effect of intensely bitter substances in pigs like the unexpected effectiveness shown by xylitol.

The masking effect of the bitter taste, for example of the quinolones, especially enrofloxacin, provided by the xylitol or its derivatives in pigs is demonstrated both if it is together in the same medical formulation and likewise if it is administered separately in differing formulations.

The preferred proportions by weight correspond to a dose of between 1 and 100 parts of xylitol to 1 part of quinolone, and especially from between 20 and 70 parts of xylitol to 1 part of quinolone, such as the enrofloxacin.

The use of xylitol can go together with any other compound of a synergic effect with it, boosting the palatability of the feed or the aqueous dissolution or reducing the proportion of xylitol to be added. Some examples of synergising products are: Saccharides and their combinations, alcohol sugars other than xylitol, acesulfame, alitame, aspartame, superaspartame, dulcine, monelline, neohesperidine dihydrochalcone, 5-nitro-2-propoxianilline, saccharine, sucralose, thaumatine, amino-acids such as arginine, glycine and tryptophan, or other sugars other than xylitol or glucosides such as those coming from *Glycyrrhiza glabra* or from *Estevia rebaudiana,* likewise the salts or compounds from any of them.

One of the compounds that has been shown to be especially useful as a synergiser on using xylitol to optimise the palatability in oral dissolutions is arginine amino-acid. The use of arginine, together with enrofloxacin or another quinolone, in a proportion by weight from one half up to double the weight of enrofloxacin or another quinolone, allowing the amount of xylitol necessary to be reduced even further in order to achieve the same masking effect.

The effect of arginine in humans to reduce the bitter effect is known, as shown in patent JP20066045117. In addition, the combined use of arginine and fluoro-quinolones in pharmaceutical preparations is not new. In this way the patent US 6,872,723 describes the manufacture of a stable injectable solution of difloxacine with L-arginine, with the intention of reducing the tissue irritation at the point of application. The patent JP2009018993 describes the masking effect on a bad taste of an antibiotic dissolution based on quinolones, in liquid form and taken orally. In this case the effect is achieved associating the quinolone with an acid (acetic and/or lactic) and an amino acid choosing from glycine, L-arginine and L-monosodium glutamate.

This present invention reveals the specific effect of the arginine in the reducing of the amount of xylitol necessary for the formulation of an oral dissolution of quinolones, especially enrofloxacin, for its application into the drinking water of pigs, and for this to be an acceptable consumption for this pig species. Unlike the patent US 6,872,723, the administration is oral and not injectable, and in regard to patent JP 2009018993, which deals solely with quinolones and human medication, this present invention is applicable to pigs, whose gustatory characteristics are clearly different from those of humans; in addition, the enrofloxacin is not used in humans but is used exclusively for veterinary purposes. Likewise, the aim described in the Japanese patent JP 2009018993 is to make the flavour of an oral dissolution more acceptable that is taken directly in the mouth, and in order to do this the quinolone acid salt is formulated with lactic and/or acetic acid, whilst in this present invention basic sodium or potassium salt is used, it is applied to the drinking water, not directly in the mouth, and is aimed at pigs.

It is important to comment that the use of only arginine, even in very high concentrations, does not succeed in masking the bitter taste of the quinolones for pigs, as and how documented later in the section "Drinking Water Trials". The inclusion of the arginine does however have an unexpected effect of significantly improving the masking effectiveness of the xylitol in pigs.

Under the terms of this present invention the xylitol can go together, apart from arginine, with and according to the final form of the product, any auxiliary excipient such as solvents and substances that make the solubility easier, preservatives, anti-oxidants, light protectors, colourings, reabsorption booster products, disintegration boosting agents, agglutinants, lubricants and stabilisers.

Therefore, this present invention includes both formulations and preparations that can contain only xylitol and equally xylitol together with one or more substances belonging to the group of quinolones, these formulations being able to go together with arginine.

Such formulations can be both dissolutions and equally solid preparations, intended to be mixed with feed such as drinking water and to be prepared with suitable excipients.

### Preferred embodiment of the invention

The invention will by preference be carried out with the administration in feed from between 1 and 100, and especially between 20 and 70 times the amount by weight of xylitol compared to the weight of quinolone, and an amount of arginine that will fluctuate from one half up to double the amount by weight of quinolone. The xylitol, quinolone and arginine can be found in the same pharmaceutical preparation, or equally in different preparations, but for their final combined use.

The derivative products of the present invention can be either in liquid or solid form and can be intended for direct application in the drinking water or for direct application to the animal feed, both as complete or as complements.

The following examples can be used to illustrate the invention, they must not be considered as being limiting on the use of the applications of same.
1.- Option of combined formulation of enrofloxacin, arginine and xylitol to be diluted in the drinking water:

| | |
|---|---|
| Xylitol | 640 g |
| Arginine | 5 g |
| Enrofloxacin | 10 g |
| NaOH | csp pH 11.00 |
| Water | csp 1000 ml |

5 g of arginine is added to 500 ml of water at room temperature and it is shaken until it completely dissolves. Next 10 g of enrofloxacin is added under agitation, after this sodium hydroxide in a 50% aqueous dissolution in an amount that is sufficient so that the enrofloxacin is completely dissolved. The dissolution is heated to 55°C, then 640 g of xylitol is added and keeping it at this temperature and continuing with the agitation, and then adding the remaining water to bring it up to 1,000 ml. The agitation continues until a clear solution is achieved, at which time the agitation is stopped and the solution is allowed to cool down to room temperature.
A 5 ml dose of this preparation per litre of water produces the required chemical therapeutic effect that allows normal levels of water to be ingested by the pigs.
2.- Option of a formula with two liquid components:
A) Xylitol Formula:

| | |
|---|---|
| Xylitol | 640 g |
| Water qsq | 1,000 ml |

The water is heated to 55° C, making the xylitol soluble. The agitation is continued until a clear and transparent dissolution is achieved.
B) Formula of enrofloxacin and arginine:

| | |
|---|---|
| Arginine | 5 g |
| Enrofloxacin | 10 g |
| Sodium Hydroxide csp | pH 11.00 |
| Water qsq | 1,000 ml |

5 g of arginine is added to 500 ml of water, agitating it until the product has dissolved. Next 10 g of enrofloxacin is added under agitation, after this sodium hydroxide in a 50% aqueous dissolution in an amount that is sufficient so that the enrofloxacin is completely dissolved. The necessary amount of water is added to bring it up to 1,000 ml.
A 5 ml dose of xylitol per litre of drinking water allows normal levels of water to be ingested that is medicated with 0.5 ml of the enrofloxacin and arginine formula (50 g of enrofloxacin and 25 g of arginine) per litre of drinking water, which corresponds to a therapeutic dosage of 4 mg per kilo of live weight.
3.- Option of a solid form of xylitol so as to administer drinking water medicated with erofloxacine, or equally animal feed medicated with enrofloxacin.
Xylitol formulation:

| | |
|---|---|
| Xylitol | 640 gr |
| Lactose qsq | 1,000 gr |

A 5 gr dose of this mixture per litre of drinking water allows normal levels of intake to be maintained when the water is medicated with 50 mg of enrofloxacin, and 25 gm of arginine, per 1 litre of drinking water as and how described in example 2, which corresponds to a therapeutic dosage of 4 mg per kg of live weight.
A 4 g dosage of this mixture per kilo of animal feed allows the complete intake of the rations when said animal feed is medicated with 0.1 g of enrofloxacin per kilo of animal feed, which corresponds to a therapeutic dosage of 4 mg per kg of live weight.
4.- Option of solid premixing so as to mix it in with the animal feed.

| | |
|---|---|
| Enrofloxacin | 25 gr |
| Xylitol | 780 gr |
| Lactose csp | 1,000 gr |

A 4 g dosage of this mixture per kilo of animal feed ensures complete intake of the animal feed ration the same as a therapeutic 4 mg dosage of enrofloxacin per kg of live weight.

### Trials in the drinking water

### A) Animals weighing 10 kg

For 9 consecutive days a water consumption trial was carried out with 140 animals each weighing 10 kg. On the first day the normal consumption of the animals was established, this being a total of 600 litres. The second and third day the drinking water was administered with a dissolution of enrofloxacin and arginine in such a way that concentrations of 0.05 g and 0.025 g, respectively were reached per litre of drinking water, together with another dissolution of xylitol in such a way that a concentration of 3.5 g of xylitol per litre of drinking water was reached. The consumption on these days was seen as being practically the same consumption as there had been if the water had not been medicated.

The fourth day a new blank test was carried out, with similar results to the preceding three days.

The fifth and sixth day the drinking water was administered on one hand with a dissolution of enrofloxacin reaching a concentration of 0.05 g per litre of drinking water, and on the other hand a dissolution of xylitol, this time without the arginine, reaching a concentration of 3.2 g per litre of drinking water. In this case with the amount of xylitol used in days 2 and 3, but without arginine, the water consumption reduced to some 70% of the amounts consumed when the arginine was present (which was days 2 and 3).

The seventh day a new blank was carried out with results that were similar to those of the first four days.

On day 8 the drinking water was administered with a dissolution of only enrofloxacin and arginine, reaching concentrations of 0.05 g and 0.025 g respectively, per litre of drinking water. An extreme drop in water consumption was noticed, when it was decided to suspend the trial from day 9, for the well being of the animals.

| DAY | TRIAL | Animals of 10 kg | Grams of enrofloxacin per litre of water | Grams of L-arginine, per litre of water | Grams of xylitol per litre of water | Consumption of water over 24 h |
|---|---|---|---|---|---|---|
| 1 | Blank | 140 | 0 | 0 | 0 | 600 litres |
| 2 | Enrofloxacin | 140 | 0,05 | 0.025 | 3.2 | 588 litres |
| 3 | Enrofloxacin | 140 | 0,05 | 0.025 | 3.2 | 593 litres |
| 4 | Blank | 140 | 0 | 0 | 0 | 597 litres |
| 5 | Enrofloxacin | 140 | 0.05 | 0 | 3.2 | 415 litres |
| 6 | Enrofloxacin | 140 | 0.05 | 0 | 3.2 | 409 litres |
| 7 | Blank | 140 | 0 | 0 | 0 | 607 litres |
| 8 | Enrofloxacin | 140 | 0.05 | 0.025 | 0 | 64 litres |
| 9 | Enrofloxacin | 140 | 0.05 | 0.025 | 0 | The trial was suspended |

### B) Animals weighing 15 and 35 kg

A similar trial was carried out as the one described for animals weighing 10 kg, with 140 animals and with equivalent results.

| DAY | Trial | Animals of 15 kg | Animals of 35 kg | Grams of Enrofloxacin per litre of water | Grams of L-arginine per litre of water | Grams of xylitol per litre of water | Consumption of water over 12 h |
|---|---|---|---|---|---|---|---|
| 1 | Blank | 140 | 70 | 0 | 0 | 0 | 450 litres |
| 2 | Enrofloxacin | 140 | 70 | 0,05 | 0.025 | 3.2 | 457 litres |
| 3 | Enrofloxacin | 140 | 70 | 0,05 | 0.025 | 3.2 | 450 litres |
| 4 | Enrofloxacin | 140 | 70 | 0,05 | 0.025 | 3.2 | 464 litres |
| 5 | Blank | 140 | 70 | 0 | 0 | 0 | 461 litres |
| 6 | Enrofloxacin | 140 | 70 | 0.05 | 0 | 3.2 | 368 litres |
| 7 | Enrofloxacin | 140 | 70 | 0.05 | 0 | 3.2 | 372 litres |
| 8 | Blank | 140 | 70 | 0 | 0 | 0 | 457 litres |
| 9 | Enrofloxacin | 140 | 70 | 0.05 | 0.025 | 0 | 38 litres |
| 10 | Enrofloxacin | 140 | 70 | 0.05 | 0.025 | 0 | The trial was suspended |

### C) Animals weighing 40 kg

A similar trial to that carried out with animals of 10 kg is described, with 350 animals and with equivalent results.

| DAY | Trial | Animals of 40 kg | Grams of enrofloxacin per litre of water | Grams of L-arginine per litre of water | Grams of xylitol per litre of water | Consumption of water over 24 h |
|---|---|---|---|---|---|---|
| 1 | Blank | 350 | 0 | 0 | 0 | 1151 litres |
| 2 | Enrofloxacin | 350 | 0,05 | 0.025 | 3.2 | 1140 litres |
| 3 | Enrofloxacin | 350 | 0,05 | 0.025 | 3.2 | 1153 litres |
| 4 | Enrofloxacin | 350 | 0,05 | 0.025 | 3.2 | 1166 litres |
| 5 | Blank | 350 | 0 | 0 | 0 | 1159 litres |
| 6 | Enrofloxacin | 350 | 0.05 | 0 | 3.2 | 792 litres |
| 7 | Enrofloxacin | 350 | 0.05 | 0 | 3.2 | 799 litres |
| 8 | Blank | 350 | 0 | 0 | 0 | 1169 litres |
| 9 | Enrofloxacin | 350 | 0.05 | 0.025 | 0 | 94 litres |
| 10 | Enrofloxacin | 350 | 0.05 | 0.025 | 0 | The trial was suspended |

### TRIALS WITH ANIMAL FEED

### Animals weighing 15 kg

### Positive control

| DAY | TRIAL | Animals of 15 kg | Grams of enrofloxacin per Kilo of animal feed | Grams of xylitol per kilo of animal feed | CONSUMPTION of animal feed in 24 h |
|---|---|---|---|---|---|
| 1 | Blank | 25 | 0 | 0 | 12 |
| 2 | Blank | 25 | 0 | 0 | 12 |

### Negative control

| DAY | TRIAL | Animals of 15 kg | Grams of enrofloxacin per kilo of animal feed | Grams of xylitol perm kilo of animal feed | CONSUMPTION of animal feed in 8 h * |
|---|---|---|---|---|---|
| 1 | Enrofloxacin | 25 | 0.1 | 0 | Nil |
| 2 | Enrofloxacin | 25 | 0.1 | 0 | Nil |

| | | | | | |
|---|---|---|---|---|---|
| (*) the trial was suspended after 8 hours in view of the complete rejection | | | | | |

### Trial 1

| DAY | TRIAL | Animals of 15 kg | g Enrofloxacin base/kg of animal feed | Xylitol Added g | CONSUMPTION K/24h |
|---|---|---|---|---|---|
| 1 | Blank | 20 | 0 | 0 | 12 |
| 2 | Enrofloxacin | 20 | 0.1 | 3.2 | 12 |
| 3 | Enrofloxacin | 20 | 0.1 | 3.2 | 12 |

### Trial 2

| DAY | TRIAL | Animals of 15 kg | g Enrofloxacin base/kg of animal feed | Xylitol Added g | CONSUMPTIONKg/24h |
|---|---|---|---|---|---|
| 1 | Blank | 20 | 0 | 0 | 12 |
| 2 | Enrofloxacin | 20 | 0.1 | 3.2 | 12 |
| 3 | Enrofloxacin | 20 | 0.1 | 3.2 | 12 |

Once having sufficiently described the nature of the invention, likewise some examples of a preferred embodiment it is placed on record that the materials, shape, size and arrangement of the elements described can be modified provided that they do not mean an alteration of the essential characteristics of the invention that are claimed below.

## Claims

1. Use of xylitol or its derivatives for the gustatory masking of chemotherapy drugs of quinolone-o-naftiridon carboxylic acid administered in animal feedstocks for pigs, alone or in the presence of arginine and accompanied by other synergising and excipient agents.

2. Use of xylitol or its derivatives according to claim 1, **characterised in that** the chemotherapy drug group of quinolone-o-naftiridon carboxylic acid is particularly enrofloxacin.

3. Use of xylitol or its derivatives and arginine, according to claim 1, **characterised in that** the chemotherapy drug group of quinolone-o-naftiridon carboxylic acid, xylitol and arginine are used with a joint proportional weight from between 1 part of the chemotherapy drug of the quinolone-o-naftiridon carboxylic acid group from 1 to 100 parts of xylitol, and from 0.5 to 2 parts of arginine.

4. Use of xylitol or its derivatives, according to claim 1, **characterised in that** the synergising agent is chosen from amongst the group of saccharides and their combinations, alcohol sugars other than xylitol, acesulfame, alitame, aspartame, superaspartame, dulcine, monelline, neohesperidine dihydrochalcone, 5-nitro-2-propoxianilline, peniatine, saccharine, sucralose, thaumatine, glucosides coming from *Glycyrrhiza glabra* or from *Estevia rebaudiana, and* amino-acids such as, glycine and tryptophan, or other sugars likewise the salts or compounds from any of them.

5. Use, according to claim 1, **characterised in that** the excipients include at least one of the following components: Solvents and substances that make the solubility easier, preservatives, anti-oxidants, protectors against the light, colourings, reabsorption booster products, disintegration booster agents, agglutinants, lubricants or stabilisers.

6. Use, according to claim 1, **characterised in that**, it includes the use of xylitol, arginine and synergises in an aqueous dissolution.

7. Use, according to claim 1, **characterised in that** it includes the use of xylitol, arginine and synergises in a solid state.
